(19) Europäisches Patentamt
European Patent Office
Office européen des brevets

(11) **EP 3 624 871 B1**

(12) **EUROPÄISCHE PATENTSCHRIFT**

(45) Veröffentlichungstag und Bekanntmachung des Hinweises auf die Patenterteilung:
**26.11.2025 Patentblatt 2025/48**

(21) Anmeldenummer: **18728539.0**

(22) Anmeldetag: **16.05.2018**

(51) Internationale Patentklassifikation (IPC):
***A61M 1/28*** (2006.01)

(52) Gemeinsame Patentklassifikation (CPC):
**A61M 1/28;** A61M 2205/18; A61M 2205/3331; A61M 2205/3334; A61M 2205/70

(86) Internationale Anmeldenummer:
**PCT/EP2018/062802**

(87) Internationale Veröffentlichungsnummer:
**WO 2018/210962 (22.11.2018 Gazette 2018/47)**

(54) **PERITONEALDIALYSEGERÄT**

PERITONEAL DIALYSIS MACHINE

APPAREIL DE DIALYSE PÉRITONÉALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.05.2017 DE 102017110577**

(43) Veröffentlichungstag der Anmeldung:
**25.03.2020 Patentblatt 2020/13**

(73) Patentinhaber: Fresenius Medical Care Deutschland GmbH
**61352 Bad Homburg (DE)**

(72) Erfinder:
• **WOLF, Klaus**
**97450 Muedesheim (DE)**
• **WABEL, Peter**
**64287 Darmstadt (DE)**

(74) Vertreter: **Lorenz Seidler Gossel Part. mbB Widenmayerstr. 23 80538 München (DE)**

(56) Entgegenhaltungen:
**DE-A1- 102014 005 122**

## Beschreibung

Die Erfindung betrifft ein Gerät für die Durchführung einer automatisierten Peritonealdialysebehandlung an einem Patienten.

Die Peritonealdialyse (PD) ist auch unter dem Begriff Bauchfelldialyse bekannt. Es gibt unterschiedliche PD-Verfahren, darunter die mit Peritonealdialysegeräten durchgeführten Verfahren der automatisierte Peritonealdialyse (APD). Bei der APD werden alle oder zumindest einige der Behandlungsschritte automatisiert durchgeführt. Dies können beispielsweise das Ein- oder Ausschalten von Pumpen, das Öffnen oder Schließen von Ventilen etc. sein.

Das Bewirken einer Strömung von Dialyselösung kann gravimetrisch, d.h. schwerkraftbedingt, und/oder mittels einer oder mehrere Pumpen erfolgen.

Die vorliegende Erfindung ist auf kein besondere Art der PD beschränkt, d.h. umfasst sowohl automatische, nicht automatische, gravimetrische sowie auch mit Pumpen arbeitende Geräte.

Bei der PD führt das Gerät dem Patienten in einer Einlaufphase über einen Katheter eine Dialyselösung in die Bauchhöhle ein. Dieser Schritt kann wie auch der Ablauf aus der Bauchhöle gravimetrisch erfolgen. Die Dialyselösung wird dann während einer Halteperiode in der Bauchhöhle belassen. Dabei können kleinmolekulare Substanzen aus dem Blut über die Kapillargefäße des Bauchfells in die Dialyselösung übertreten, da ein Konzentrationsgefälle herrscht. Ferner kann dem Körper auf diesem Wege auch Wasser entzogen werden, sofern die Dialyselösung einen höheren Gehalt an osmotisch aktiven Substanzen aufweist als das Blut. Nach Ende der Halteperiode entfernt das Gerät die mit ausgeschiedenen Substanzen angereicherte und mithin verbrauchte Dialyselösung in einer Ablaufphase über den Katheter wieder aus der Bauchhöhle. Der Flüssigkeitstausch kann gravimetrisch oder aktiv anhand einer Pumpe erfolgen.

Der Zyklus aus Einlaufphase, Halteperiode und Ablaufphase wird bei typischen Verfahrensführungen mehrmals wiederholt, beispielsweise in der Nacht, während der Patient schläft. Eine neue Einlaufphase wird immer dann gestartet, wenn das Gerät feststellt, dass die Ablaufphase beendet ist, d.h., dass die verbrauchte Dialyseflüssigkeit vollständig aus der Bauchhöhle des Patienten abgelaufen ist.

Im Stand der Technik ist es bekannt, im Interesse einer besseren Planungssicherheit für Betreuer und Patienten eine Abschätzung der Gesamtbehandlungsdauer einer Pertionealdialyse-Behandlung umfassend mehrere Zyklen bereitzustellen. Beispielsweise wird in der DE 10 2014 005 122 A1 offenbart, die Gesamtbehandlungsdauer dadurch abzuschätzen, indem die ideale Behandlungsdauer als Ausgangspunkt genommen wird und in bestimmter Weise erhöht wird. Unter anderem sind Verzögerungsfaktoren für die Einlaufdauer und die Ablaufdauer von Dialyseflüssigkeit zum bzw. vom Patienten vorgesehen, die ein nicht ideales Verhalten des Katheters wiederspiegeln wollen (beispielsweise ein Faktor bzw. Multiplikator von 1,5). Bisher wurden solche Verzögerungsfaktoren jedoch lediglich geschätzt bzw. patientenunspezifisch eingestellt, wodurch die Abschätzung der Gesamtbehandlungsdauer zwar verbessert werden konnte, das Ergebnis jedoch weiterhin mit erheblichen Unsicherheiten belegt war. Eine etwaige fortlaufende Verschlechterung der Katheterqualität durch Zusetzen mit Fibrin oder eine Lageänderung des Katheters innerhalb des Patienten (innerhalb der Bauchhöle bzw. Douglas-Raum) wurde bisher nicht berücksichtigt.

Aufgabe der Erfindung ist es, ein Peritonealdialysegerät bereitzustellen, mit dem eine zuverlässigere Schätzung der Gesamtbehandlungsdauer vorgenommen werden kann.

Vor diesem Hintergrund betrifft die Erfindung ein Peritonealdialysegerät für die Durchführung einer automatisierten Peritonealdialysebehandlung mit sich wiederholenden Zyklen, die Zyklen umfassend eine Einlaufphase, eine Halteperiode und eine Ablaufphase für die Dialyseflüssigkeit, wobei das Gerät eine Steuereinheit und eine Messvorrichtung zur Bestimmung des Einlauf- und/oder Ablaufverhaltens der Dialyseflüssigkeit von einem Patienten aufweist. Erfindungsgemäß ist vorgesehen, dass die Steuereinheit ausgebildet ist, auf der Grundlage der über mehrere Einlauf -bzw. Ablaufphasen gesammelten Messwerte einen Verzögerungsfaktor zu bestimmen, der eine theoretische Ein- und/oder Ablaufdauer mit der tatsächlichen Ein- und/oder Ablaufdauer von Dialyseflüssigkeit zum bzw. vom Patienten ins Verhältnis setzt.

Die Erfindung zielt also auf die Bestimmung eines patientenindividuellen Verzögerungsfaktors ab. Dieser Verzögerungsfaktor kann beispielsweise der anhand einer Mehrzahl an gemessenen Werten bzw. Werteverläufen (Kurven) ermittelt werden, die in einer bestimmten Anzahl an vorangegangenen Zyklen bzw. Behandlungen gemessen wurden. Es kann sich also um einen patientenindividuellen Mittelwert handeln.

In einer Ausführungsform ist vorgesehen, dass es sich bei dem Gerät um ein gravimetrisch arbeitendes Gerät handelt. Beispielsweise können ein oder mehrere Ventile vorgesehen und die Steuereinheit so ausgebildet sein, dass das oder ein Ventil nach der Haltephase und vor Beginn der Ablaufphase geöffnet wird, um einen gravimetrischen Ablauf von Dialyseflüssigkeit vom Patienten zu ermöglichen, und/oder dass das oder ein Ventil nach der Ablaufphase und vor Beginn der Einlaufphase geöffnet wird, um einen gravimetrischen Einlauf von Dialyseflüssigkeit zum Patienten zu ermöglichen.

In einer Ausführungsform ist vorgesehen, dass das Gerät eine Dialysepumpe aufweist, die ausgebildet ist, um Dialyseflüssigkeit vom und/oder zum Patienten zu pumpen. Es kann sich also um ein aktiv förderndes Gerät handeln, in dem die Steuereinheit so ausgebildet ist, dass dem Patienten anhand der Dialysepumpe während der Ablaufphase Dialyseflüssigkeit entzogen und/oder dem Patienten anhand der Pumpe während der Einlaufphase Dialyseflüssigkeit zugeführt wird.

In einer Ausführungsform ist vorgesehen, dass die Messvorrichtung ausgebildet ist, die Flussrate der Dialyseflüssigkeit zu bestimmen. Es kann also ein Flusssensor vorgesehen sein, anhand dessen der zeitliche Verlauf der Flussrate während der Einlauf- und/oder Ablaufphase bestimmt und in der Steuereinheit aufgezeichnet werden kann. In dieser Ausführungsform kann vorgesehen sein, dass die Steuereinheit ausgebildet ist, den Verzögerungsfaktor auf der Grundlage der über mehrere Einlauf -bzw. Ablaufphasen gesammelten Messwerte für die Flussraten zu bestimmen. Der zeitliche Verlauf der Flussrate bzw. die Flussrate selbst kann auch gravimetrisch durch das Wiegen der Lösung bzw. eines Beutels oder eines sonstigen Gefäßes mittels einer Waage zu zwei unterschiedlichen Zeitpunkten erfolgen. Auch daraus kann auf die Flussrate geschlossen bzw. diese berechnet werden.

In einer Ausführungsform ist vorgesehen, dass die Messvorrichtung ausgebildet ist, einen hydrostatischen Druck in der Leitung oder eine Druckdifferenz an einer Dialysepumpe zu bestimmen. Es kann also ein Drucksensor vorgesehen sein, anhand dessen der Verlauf des hydrostatischen Drucks in der Leitung, insbesondere bei gravimetrischen Systemen, oder der Druckabfall an einer Dialysepumpe, bei aktiven, d.h. wenigstens eine Pumpe aufweisenden Systemen, während der Einlauf- und/oder Ablaufphase bestimmt und in der Steuereinheit aufgezeichnet werden kann. **In** dieser Ausführungsform kann vorgesehen sein, dass die Steuereinheit ausgebildet ist, den Verzögerungsfaktor auf der Grundlage der über mehrere Einlauf -bzw. Ablaufphasen gesammelten Messwerte für die Drücke bzw. Druckdifferenzen zu bestimmen. Die Druckdifferenzen können in der Steuereinheit als Funktionen der Zeit oder auch als Funktionen des Füllvolumens aufgezeichnet werden. Insofern kann das Gerät eine Messvorrichtung für sowohl Flussrate als auch Druck bzw. Druckdifferenz aufweisen, und es kann vorgesehen sein, dass der Verzögerungsfaktor auf der Grundlage beider dieser Messwerte bestimmt wird.

**In** einer Ausführungsform ist vorgesehen, dass die Steuereinheit ausgebildet ist, eine Schätzung der Gesamtbehandlungsdauer für eine bestimmte Verschreibung auszugeben, wobei in der Erstellung der Schätzung eine theoretische Einlauf- und/oder Ablaufdauer mit dem Verzögerungsfaktor multipliziert wird. Beispielsweise kann vorgesehen sein, dass die Bestimmung der idealen Gesamtbehandlungsdauer die Multiplikation einer idealen Zyklusdauer mit der Anzahl der Zyklen umfasst. Die Bestimmung der realen Gesamtbehandlungsdauer umfasst entsprechend die Multiplikation einer realen Zyklusdauer mit der Anzahl der Zyklen. Die ideale Zyklusdauer bestimmt sich aus der Summe der idealisierten Dauer der Einlaufphase, der idealisierten Dauer der Haltephase und der idealisierten Dauer der Ablaufphase. Die reale Zyklusdauer bestimmt sich entsprechend aus der Summe der realen Dauer der Einlaufphase, der realen Dauer der Haltephase und der realen Dauer der Ablaufphase. Die reale Dauer der Einlaufphase kann erfindungsgemäß nun durch Multiplikation der idealisierten Dauer der Einlaufphase mit dem Verzögerungsfaktor erhalten werden. Entsprechend kann die reale Dauer der Ablaufphase erfindungsgemäß durch Multiplikation der idealisierten Dauer der Ablaufphase mit dem Verzögerungsfaktor erhalten werden.

In einer Ausführungsform ist vorgesehen, dass die Steuereinheit so ausgebildet ist, dass ein Signal ausgegeben wird, wenn der Verzögerungsfaktor einen Schwellenwert übersteigt. Zu diesem Zweck kann vorgesehen sein, dass das Gerät eine Signaleinheit oder eine Schnittstelle zur Kommunikation mit einer externen Signaleinheit aufweist. Geeignete Signale umfassen beispielsweise optische Signale, Tonsignale oder Vibrationssignale. Auf der Grundlage einer Signalausgabe kann beispielsweise entschieden werden, ob es Sinn machen könnte, angesichts der zeitlichen Umstände die Verschreibung zu ändern. Ferner kann beispielsweise entschieden werden, ob es Sinn machen könnte, den Katheter zu tauschen. Eine Auswertung auf der Grundlage von graphischen oder tabellarischen Aufzeichnungen in einer geeigneten Software wird so ermöglicht.

In einer Ausführungsform ist vorgesehen, dass die Steuereinheit so ausgebildet ist, dass ein Behandlungsparameter modifiziert wird, wenn der Verzögerungsfaktor einen bestimmten Schwellenwert übersteigt. Es kann also vorgesehen sein, Behandlungsparameter unter Heranziehung des Verzögerungsfaktors besser an die vorliegenden Realbedingungen anzupassen.

In einer Ausführungsform ist vorgesehen, dass es sich bei dem modifizierten Behandlungsparameter um ein Kriterium zur Feststellung des Endes einer Ablaufphase handelt, vorzugsweise eine minimale Abflussrate. Es kann vorgesehen sein, dass die Steuereinheit ausgebildet ist, um die Feststellung, ob eine Ablaufphase beendet ist und eine neue Einlaufphase beginnen kann, anhand voreingestellter Kriterien zu treffen, beispielsweise anhand eines erreichten Minimalablaufvolumens und/oder anhand der Unterschreitung einer bestimmten Flussrate.

In einer Ausführungsform ist vorgesehen, dass es sich bei dem modifizierten Behandlungsparameter um die Pumprate einer Dialysepumpe handelt, die ausgebildet ist, um Dialyseflüssigkeit vom und/oder zum Patienten zu pumpen. Dabei kann vorgesehen sein, dass ein Ausgangswert für die Pumprate modifiziert wird, beispielsweise die Pumprate, die zu Beginn einer Ablaufphase eingestellt wird. Ferner kann vorgesehen sein, dass ein Verlaufsprofil für die Pumprate modifiziert wird, beispielsweise die Änderung der anfänglichen Pumprate im Verlauf einer Ablaufphase.

Des Weiteren kann wenigstens ein Speicher vorgesehen sein, in dem der Verzögerungsfaktor im Behandlungsprotokoll abgelegt ist, um eine Weiterverarbeitung, vorzugsweise eine graphische Aufarbeitung des Vezögerungsfaktors über mehrere Behandlungen hinweg zu ermöglichen. Der Speicher kann einen Bestandteil des Gerätes darstellen oder auch als externer Speicher ausgeführt sein.

Ferner umfasst die Erfindung ein Verfahren zur Durchführung einer Peritonealdialyse unter Verwendung eines erfindungsgemäßen Peritonealdialysegeräts, wobei auf der Grundlage der über mehrere Einlauf -bzw. Ablaufphasen gesammelten Messwerte ein Verzögerungsfaktor bestimmt wird, der eine theoretische Ein- und/oder Ablaufdauer mit der tatsächlichen Ein- und/oder Ablaufdauer von Dialyseflüssigkeit zum bzw. vom Patienten ins Verhältnis setzt. Vorteilhafte Ausgestaltungen des Verfahrens ergeben sich aus der obigen Beschreibung der Ausbildung der Steuereinheit im erfindungsgemäßen Peritonealdialysegerät.

Weitere Einzelheiten und Vorteile der Erfindung ergeben sich aus dem nachfolgend anhand der Figuren erklärten Ausführungsbeispiel. In den Figuren zeigen:

Figur 1: einen möglichen Verlauf der Katheterqualität im Verlauf mehrerer Behandlungen;

Figur 2: einen anderen möglichen Verlauf der Katheterqualität im Verlauf mehrerer Behandlungen; und

Figur 3: mögliche zeitliche Verläufe der Abflussrate (Volumen/Zeit) von Dialyseflüssigkeit aus der Bauchhöhle während der Ablaufphase.

Gemäß Ausführungsbeispiel ist ein Peritonealdialysegerät vorgesehen, welches für die Durchführung einer automatisierten Peritonealdialysebehandlung mit sich wiederholenden Zyklen umfassend eine Einlaufphase, eine Halteperiode und eine Ablaufphase für die Dialyseflüssigkeit bestimmt ist. Das Gerät umfasst eine Steuereinheit, eine Messvorrichtung zur Bestimmung der Ein- und Ablaufrate der Dialyseflüssigkeit von einem Patienten und eine Dialysatpumpe zum Fördern von Dialyseflüssigkeit an den Patienten und vom Patienten.

In der Steuereinheit ist ein Algorithmus hinterlegt, anhand dessen eine Bestimmung der realen Gesamtbehandlungsdauer ($\Delta t_{Gesamt,real}$) vorgenommen werden kann. Der Algorithmus beruht auf einer Multiplikation einer realen Zyklusdauer ($\Delta t_{Zyklus,real}$) mit der Anzahl der Zyklen (n), ergänzt um eine reale Dauer einer initialen Ablaufphase ($\Delta t_{Init,real}$) und eine reale Dauer einer finalen Einlaufphase ($\Delta t_{Fin,real}$).

$$\Delta t_{Gesamt,real} = \Delta t_{Zyklus,real} \times n + \Delta t_{Init,real} + \Delta t_{Fin,real}$$

Die reale Zyklusdauer ($\Delta t_{Zyklus,real}$) bestimmt sich aus der Summe der realen Einlaufdauer ($\Delta t_{Ein,real}$), der realen Haltedauer ($\Delta t_{Halte,real}$) und der realen Ablaufdauer ($\Delta t_{Ab,real}$).

$$\Delta t_{Zyklus,real} = \Delta t_{Ein,real} + \Delta t_{Halte,real} + \Delta t_{Ab,real}$$

Die vorliegende Erfindung beschäftigt sich mit der genauen Bestimmung der realen Einlaufdauer ($\Delta t_{Ein,real}$), realen Ablaufdauer ($\Delta t_{Ab,real}$), der realen Dauer der initialen Ablaufphase ($\Delta t_{Init,real}$) und der realen Dauer der finalen Einlaufphase ($\Delta t_{Fin,real}$). Die Dauer dieser realen Phasen wird erfindungsgemäß aus der Multiplikation der korrespondierenden idealen Dauern ($\Delta t_{Ein,ideal}$, $\Delta t_{Ab,ideal}$, $\Delta t_{Init,ideal}$ und $\Delta t_{Fin,ideal}$), die sich bei bekannter Gerätekonfiguration errechnen lassen, mit einem Verzögerungsfaktor F. Es sind unterschiedliche Verzögerungsfaktoren für Einlaufvorgänge ($\Delta t_{Ein}$, $\Delta t_{Fin}$), und die Ablaufvorgänge ($\Delta t_{Ab}$, $\Delta t_{Init}$), vorgesehen, nämlich die Faktoren $F_{Ab}$ und $F_{Ein}$.

$$\Delta t_{Ein,real} = \Delta t_{Ein,ideal} \times F_{Ein}$$

$$\Delta t_{Ab,real} = \Delta t_{Ab,ideal} \times F_{Ab}$$

$$\Delta t_{Init,real} = \Delta t_{Init,ideal} \times F_{Ab}$$

$$\Delta t_{Fin,real} = \Delta t_{Fin,ideal} \times F_{Ein}$$

Die Verzögerungsfaktoren $F_{Ab}$ und $F_{Ein}$ werden von der Steuereinheit anhand einer Mehrzahl an gemessenen Verläufen der Ab- bzw. Einlaufraten ermittelt, die in einer bestimmten Anzahl an vorangegangenen Zyklen gemessen wurden (beispielsweise 20 Zyklen). Es handelt sich daher um einen patientenindividuellen Mittelwert, der repräsentativ für die Katheterqualität ist.

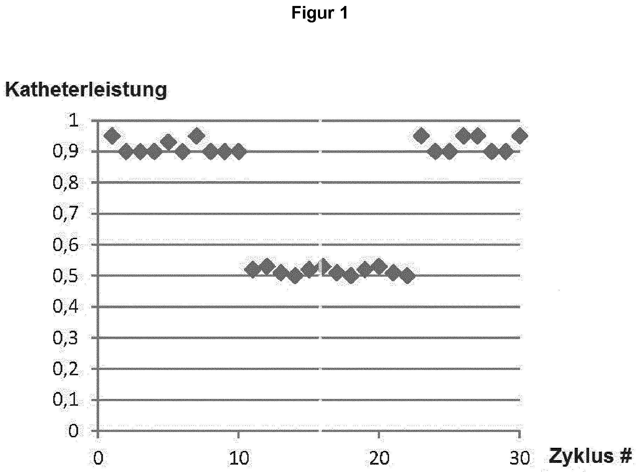

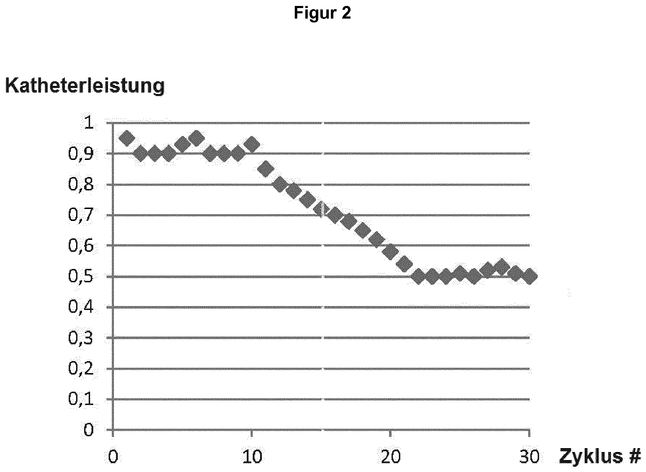

In Figuren 1 und 2 werden mögliche Verläufe der Katheterqualität im Verlauf mehrerer Behandlungen dargestellt. Die Abszisse bezeichnet die laufende Nummer eines Zyklus aus Einlaufphase, Haltephase und Ablaufphase. Die Ordinate bezeichnet eine Größe repräsentativ für die Katheterleistung, die beispielsweise anhand des Flusses bei bestimmter Druckdifferenz bestimmt werden kann. Der Wert 1 steht für ideale Katheterleistung, Werte unter 1 für entsprechend verminderte Katheterleistung. In der Figur 1 ist nach einer bestimmten Anzahl an Zyklen ein plötzlicher, signifikanter Abfall der Katheterleistung zu beobachten, was beispielsweise durch eine plötzliche Änderung der Positionierung des Katheters in der Bauchhöhle des Patienten bedingt sein kann. In Figur 2 wird ein schleichender Abfall der Katheterleistung illustriert, wie er beispielsweise durch ein fortlaufendes Zusetzen des Katheters mit Fibrin beobachtet werden kann.

Im Gerät gemäß Ausführungsbeispiel ist die Steuereinheit ferner ausgebildet, ein Signal auszugeben, wenn ein Verzögerungsfaktor $F_{Ab}$ oder $F_{Ein}$ einen in der Steuereinheit hinterlegten Schwellenwert übersteigt. Zu diesem Zweck umfasst das Gerät eine Schnittstelle zur Kommunikation mit einem externen Rechner. Auf der Grundlage einer Signalausgabe kann beispielsweise entschieden werden, ob es Sinn machen könnte, angesichts der zeitlichen Umstände die Verschreibung zu ändern. Ferner kann beispielsweise entschieden werden, ob es Sinn machen könnte, den Katheter zu tauschen, die Position des Katheters zu prüfen, zu korrigieren oder ggf. den Kathether zu spülen. Die Auswertung erfolgt auf der Grundlage von graphischen und tabellarischen Aufzeichnungen in einer geeigneten Software.

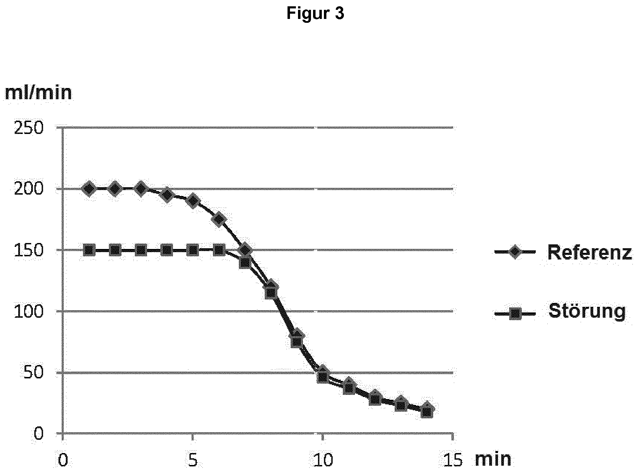

Die Steuereinheit ist ferner so ausgebildet, dass der als Kriterium zur Feststellung des Endes einer Ablaufphase herangezogene Wert für eine Abflussrate am Ende der Ablaufphase um einen bestimmten Betrag nach unten korrigiert wird, wenn der Verzögerungsfaktor $F_{Ab}$ in der Steuereinheit hinterlegten Schwellenwert übersteigt. Figur 3 zeigt zeitliche Verläufe der Abflussrate in einem Peritionealdialysegerät. Die Kurve "Referenz" zeigt einen zeitlichen Verlauf der Abflussrate im Normalfall. Die Abflussrate stellt sich anfänglich bei etwa 200 ml/min ein und nimmt ab etwa Minute 5 aufgrund des geringer werdenden hydrostatischen Drucks ab. Die Kurve "Störung" zeigt einen zeitlichen Verlauf der Abflussrate bei beeinträchtigter Katheterleistung. Die Abflussrate stellt sich anfänglich nur mehr bei etwa 150 ml/min ein. Auch hier wird eine Abnahme aber nach etwa Minute 5 beobachtet, sodass nach etwa 10 Minuten wesentlich weniger Flüssigkeit abgelaufen ist. Unter Ausbleiben geeigneter Gegenmaßnahmen hätte dies die Konsequenz einer schlechteren Behandlungsqualität und einer längeren Behandungsdauer. Die Korrektur des Schwellenwerts steuert diesem Problem entgegen.

Ferner werden auch der Ausgangswert und der Änderungsverlauf der Rate der Dialysepumpe in der Einlaufphase oder der Auslaufphase verändert, wenn der Verzögerungsfaktor $F_{Ab}$ oder $F_{Ein}$ in der Steuereinheit hinterlegten Schwellenwert übersteigt. So kann dem Problem einer abfallenden Behandlungsqualität zusätzlich entgegengewirkt werden.

Vorteile der erfindungsgemäßen Lösung umfassen beispielsweise die Möglichkeit einer zuverlässigeren Schätzung der Gesamtbehandlungsdauer einer Peritonealdialysebehandlung bei gegebener Verschreibung. Ferner ermöglicht die Erfindung die Qualität des Katheters über den Verlauf mehrerer Behandlungen zu beobachten und auch beispielsweise graphisch zu visualisieren. Eine Früherkennung von Veränderungen am Katheter ist möglich. Die Behandlungsparameter und/oder Systemparameter können in einer Ausführungsform der Erfindung besser an eine individuelle Situation bzw. an einen individuellen Patienten angepasst werden. Auch die Notwendigkeit einer Korrektur der Verschreibung kann mit Hilfe der erfindungsgemäßen Lösung in einer Ausführungsform erkannt werden. Änderungen in der Verfassung des Patienten können erkannt werden. Benachrichtigungen und Rückmeldungen an den Patienten betreffend verschiedene Verhaltensschemata können verbessert werden.

**Patentansprüche**

1.   Peritonealdialysegerät für die Durchführung einer Peritonealdialysebehandlung mit sich wiederholenden Zyklen, die Zyklen umfassend eine Einlaufphase, eine Halteperiode und eine Ablaufphase für die Dialyseflüssigkeit, wobei das Gerät eine Steuereinheit und eine Messvorrichtung zur Bestimmung des Einlauf- und/oder Ablaufverhaltens der Dialyseflüssigkeit von einem Patienten aufweist,
**dadurch gekennzeichnet,**
**dass** die Steuereinheit ausgebildet ist, auf der Grundlage der über mehrere Einlauf - und/oder Ablaufphasen gesammelten Messwerte einen Verzögerungsfaktor zu bestimmen, der eine theoretische Ein- und/oder Ablaufdauer mit der tatsächlichen Ein- und/oder Ablaufdauer von Dialyseflüssigkeit zum bzw. vom Patienten ins Verhältnis setzt und dass die Steuereinheit so ausgebildet ist, dass ein Behandlungsparameter und/oder ein Systemparameter modifiziert wird, wenn der Verzögerungsfaktor einen bestimmten Schwellenwert übersteigt.

2.   Peritonealdialysegerät nach Anspruch 1, **dadurch gekennzeichnet, dass** es sich bei dem Gerät um ein gravimetrisch arbeitendes Gerät handelt.

3.   Peritonealdialysegerät nach Anspruch 1, **dadurch gekennzeichnet, dass** das Gerät eine Dialysepumpe aufweist, die ausgebildet ist, um Dialyseflüssigkeit vom und/oder zum Patienten zu pumpen.

4. Peritonealdialysegerät nach einem der vorhergehenden Ansprüche, **dadurch gekennzeichnet, dass** die Messvorrichtung ausgebildet ist, die Flussrate der Dialyseflüssigkeit zu bestimmen.

5. Peritonealdialysegerät nach einem der vorhergehenden Ansprüche, **dadurch gekennzeichnet, dass** die Messvorrichtung ausgebildet ist, einen hydrostatischen Druck in der Leitung oder eine Druckdifferenz an einer Dialysepumpe eines nicht rein gravimetrisch arbeitenden Peritonaldialysegeräts zu bestimmen.

6. Peritonealdialysegerät nach einem der vorhergehenden Ansprüche, **dadurch gekennzeichnet, dass** die Steuereinheit ausgebildet ist, eine Schätzung der Gesamtbehandlungsdauer für eine bestimmte Verschreibung auszugeben, wobei in der Erstellung der Schätzung eine theoretische und/oder berechnete Einlauf- und/oder Ablaufdauer mit dem Verzögerungsfaktor multipliziert wird.

7. Peritonealdialysegerät nach einem der vorhergehenden Ansprüche, **dadurch gekennzeichnet, dass** die Steuereinheit so ausgebildet ist, dass ein Signal ausgegeben wird, wenn der Verzögerungsfaktor einen Schwellenwert übersteigt.

8. Peritonealdialysegerät nach Anspruch 1, **dadurch gekennzeichnet, dass** es sich bei dem modifizierten Behandlungsparameter um ein Kriterium zur Feststellung des Endes einer Ablaufphase handelt, vorzugsweise eine minimale Abflussrate.

9. Peritonealdialysegerät nach Anspruch nach einem der vorhergehenden Ansprüche, **dadurch gekennzeichnet, dass** es sich bei dem modifizierten Behandlungsparameter um die Pumprate einer Dialysepumpe handelt, die ausgebildet ist, um Dialyseflüssigkeit vom und/oder zum Patienten zu pumpen.

10. Peritonealdialysegerät nach einem der vorhergehenden Ansprüche, **dadurch gekennzeichnet, dass** wenigstens ein Speicher vorgesehen ist, in dem der Verzögerungsfaktor im Behandlungsprotokoll abgelegt ist, um eine Weiterverarbeitung, vorzugsweise eine graphische Aufarbeitung des Vezögerungsfaktors über mehrere Behandlungen hinweg zu ermöglichen.

## Claims

1. Peritoneal dialysis machine for carrying out a peritoneal dialysis treatment with recurring cycles, the cycles comprising an inflow phase, a dwell period and a drainage phase for the dialysis fluid, the machine having a control unit and a measurement apparatus for determining the inflow and/or drainage behavior of the dialysis fluid to and/or from a patient,
**characterized in that**
the control unit is configured to determine, on the basis of the measured values collected over a plurality of inflow phases and/or drainage phases, a time delay factor that relates a theoretical inflow and/or drainage duration to the actual inflow and/or drainage duration of the dialysis fluid to and/or from the patient, and **in that** the control unit is configured to modify a treatment parameter and/or a system parameter when the time delay factor exceeds a determined threshold value.

2. Peritoneal dialysis machine in accordance with claim 1, **characterized in that** the machine is a gravimetrically working machine.

3. Peritoneal dialysis machine in accordance with claim 1, **characterized in that** the machine has a dialysis pump that is configured to pump dialysis fluid from and/or to the patient.

4. Peritoneal dialysis machine in accordance with any one of the preceding claims, **characterized in that** the measurement apparatus is configured to determine the flow rate of the dialysis fluid.

5. Peritoneal dialysis machine in accordance with any one of the preceding claims, **characterized in that** the measurement apparatus is configured to determine a hydrostatic pressure in the line or a pressure difference at a dialysis pump of a peritoneal dialysis machine not working purely gravimetrically.

6. Peritoneal dialysis machine in accordance with any one of the preceding claims, **characterized in that** the control unit is configured to output an estimate of the total treatment duration for a specific prescription, with a theoretical and/or calculated inflow duration and/or drainage duration being multiplied by the time delay factor in establishing the estimate.

7. Peritoneal dialysis machine in accordance with any one of the preceding claims, **characterized in that** the control unit is configured to output a signal when the time delay factor exceeds a threshold value.

8. Peritoneal dialysis machine in accordance with claim 1, **characterized in that** the modified treatment parameter is a criterion for fixing the end of a drainage phase, preferably a minimal outflow rate.

9. Peritoneal dialysis machine in accordance with any one of the preceding claims, **characterized in that** the modified treatment parameter is the pumping rate of a dialysis pump that is configured to pump dialysis fluid from and/or to the patient.

10. Peritoneal dialysis machine in accordance with any one of the preceding claims, **characterized in that** at least one memory is provided in which the time delay factor is stored in the treatment protocol to enable a further processing, preferably a graphical processing of the time delay factor over a plurality of treatments.


**Revendications**

1. Appareil de dialyse péritonéale pour effectuer un traitement de dialyse péritonéale avec des cycles répétitifs, les cycles comprenant une phase d'entrée, une période de maintien et une phase de sortie pour le liquide de dialyse, l'appareil présentant une unité de commande et un dispositif de mesure pour déterminer le comportement d'entrée et/ou de sortie du liquide de dialyse d'un patient,
   **caractérisé en ce que**
   l'unité de commande est réalisée pour déterminer, sur la base des valeurs mesurées collectées sur plusieurs phases d'entrée et/ou de sortie, un facteur de retard qui met en relation une durée d'entrée et/ou de sortie théorique avec la durée d'entrée et/ou de sortie réelle du liquide de dialyse vers ou depuis le patient, et **en ce que** l'unité de commande est réalisée de manière à modifier un paramètre de traitement et/ou un paramètre de système lorsque le facteur de retard dépasse une valeur seuil déterminée.

2. Appareil de dialyse péritonéale selon la revendication 1, **caractérisé en ce que** l'appareil consiste en un appareil fonctionnant par gravimétrie.

3. Appareil de dialyse péritonéale selon la revendication 1, **caractérisé en ce que** l'appareil présente une pompe de dialyse qui est réalisée pour pomper le liquide de dialyse depuis et/ou vers le patient.

4. Appareil de dialyse péritonéale selon l'une quelconque des revendications précédentes, **caractérisé en ce que** le dispositif de mesure est réalisé pour déterminer le débit du liquide de dialyse.

5. Appareil de dialyse péritonéale selon l'une quelconque des revendications précédentes, **caractérisé en ce que** le dispositif de mesure est réalisé pour déterminer une pression hydrostatique dans la conduite ou une différence de pression au niveau d'une pompe de dialyse d'un appareil de dialyse péritonéale ne fonctionnant pas uniquement par gravimétrie.

6. Appareil de dialyse péritonéale selon l'une quelconque des revendications précédentes, **caractérisé en ce que** l'unité de commande est réalisée pour fournir une estimation de la durée totale du traitement pour une prescription déterminée, une durée d'entrée et/ou de sortie théorique et/ou calculée étant multipliée par le facteur de retard lors de l'établissement de l'estimation.

7. Appareil de dialyse péritonéale selon l'une quelconque des revendications précédentes, **caractérisé en ce que** l'unité de commande est réalisée pour émettre un signal lorsque le facteur de retard dépasse une valeur seuil.

8. Appareil de dialyse péritonéale selon la revendication 1, **caractérisé en ce que** le paramètre de traitement modifié consiste en un critère pour établir la fin d'une phase de sortie, de préférence un débit minimal.

9. Appareil de dialyse péritonéale selon l'une quelconque des revendications précédentes, **caractérisé en ce que** le paramètre de traitement modifié consiste en le taux de pompage d'une pompe de dialyse qui est réalisée pour pomper le liquide de dialyse depuis et/ou vers le patient.

10. Appareil de dialyse péritonéale selon l'une quelconque des revendications précédentes, **caractérisé en ce qu'**au moins une mémoire est prévue, dans laquelle le facteur de retard est enregistré dans le protocole de traitement, afin de permettre un traitement ultérieur, de préférence un traitement graphique du facteur de retard sur plusieurs traitements.

**Figur 1**

Katheterleistung

**Figur 2**

## Katheterleistung

**Figur 3**

**IN DER BESCHREIBUNG AUFGEFÜHRTE DOKUMENTE**

**In der Beschreibung aufgeführte Patentdokumente**

- DE 102014005122 A1